# EUROPEAN PATENT SPECIFICATION

(11) **EP 3 672 655 B1**
(45) Date of publication and mention of the grant of the patent: **19.01.2022**
(21) Application number: 18765296.1
(22) Date of filing: 23.08.2018
(51) Int. Cl.: A61L 29/08, A61L 29/10, A61L 29/16, A61L 31/08, A61L 31/10, A61L 31/16, A61L 15/18, A61L 15/20, A61L 15/22, A61L 15/42, A61L 15/44, A61L 15/60, A61L 26/00

(54) **BIOMATERIAL AND METHODS OF MAKING AND USING SAID BIOMATERIAL**
BIOMATERIAL UND VERFAHREN ZUR HERSTELLUNG UND VERWENDUNG DES BIOMATERIALS
BIOMATÉRIAU ET PROCÉDÉS DE FABRICATION ET D'UTILISATION DUDIT BIOMATÉRIAU

(30) Priority: 24.08.2017 US 201762549811 P
(43) Date of publication of application: 01.07.2020
(73) Proprietor: KCI USA, Inc., San Antonio, TX 78265 (US); KCI Licensing, Inc., San Antonio, TX 78265 (US); Systagenix Wound Management, Limited, Gatwick Airport, West Sussex RH6 0PA (GB)
(72) Inventor: BOURDILLON, Katie, Leeds Cookridge LS16 6PB (GB); DELURY, Craig, Gargrave North Yorkshire BD23 3SS (GB); LOCKE, Christopher Brian, Bournemouth Dorset BH9 3SD (GB); WAITE, Alexander, Cowling Keighley BD22 0FN (GB)
(74) Representative: Simmons & Simmons
(86) International application number: PCT/US2018/047717
(87) International publication number: WO 2019/040729

(56) References cited:
- EP-A2- 0 901 795
- WO-A1-2016/120620
- GB-A- 2 392 913
- GB-A- 2 425 474

## Description

### TECHNICAL FIELD

The present disclosure relates generally to biomaterials, methods of making the biomaterials and methods of using the biomaterials, more particularly, but without limitation, use of the biomaterials in wound dressings and for methods of wound therapy.

### BACKGROUND

A wide variety of materials and devices, generally characterized as "dressings," are known in the art for use in treating a wound or other disruption of tissue. Such wounds may be the result of trauma, surgery, or disease, and may affect skin or other tissues. In general, dressings may control bleeding, absorb wound exudate, ease pain, assist in debriding the wound, protect wound tissue from infection, or otherwise promote healing and protect the wound from further damage.

Infections can prevent wound healing and lead to chronic wounds due to the presence of bacteria and bacterial products, such as endotoxins and metalloproteinases, in the wound, which disrupt wound healing. Wound infections, if untreated, can result in tissue loss, systemic infections, septic shock and death. Thus, reduction in the number of bacteria is important in wound therapy. Moreover, in addition to vegetative or free-floating bacteria present in a wound, bacterial biofilms may also form in a wound presenting further challenges in wound therapy, particularly chronic wounds. A biofilm is an association of microorganisms, e.g., single or multiple species, that can adhere to a surface forming three-dimensional microbial communities, which can have coordinated multi-cellular behavior. Typically, biofilms can produce extracellular polysaccharides thereby forming an extracellular matrix in which the bacteria are embedded. The ability of bacteria to form these complex biofilms can impede a host's defense mechanisms against pathogens. For example, it is believed that the extracellular matrix surrounding the bacterial cells can provide a barrier, which can hinder or prevent penetration by the biocides. As such, biofilms often display a tolerance or recalcitrance to antimicrobial treatment. Thus, while known antimicrobial compositions, e.g., as part of a wound dressing, may be effective in reducing vegetative or free-flowing bacteria in vitro, those same antimicrobial compositions are ineffective against the same bacteria when present in a biofilm. GB 2425474 A concerns photostable wound dressing materials and methods of production thereof. EP 0901795 A2 concerns buffered wound dressing materials. WO 2016/120620 A1 concerns citric acid containing wound dressings for disrupting biofilms. GB 2392913 A concerns a complex of an anionic polysaccharide with silver. Therefore, a need remains for improved compositions having improved effectiveness against biofilms.

### BRIEF SUMMARY

The present invention provides a biomaterial for use according to claim 1. Further preferred features are provided according to the dependent claims. Illustrative embodiments are also provided to enable a person skilled in the art to make and use the claimed subject matter.

In one aspect, the present disclosure provides a biomaterial e.g., in film form, in sponge form, etc. The biomaterial, e.g., in film form, in sponge form, etc., comprises collagen, citric acid (an antimicrobial agent) and oxidized regenerated cellulose (ORC). The citric acid may be present in a concentration > 20 mM. The biomaterial, e.g., in film form, in sponge form, etc., may further comprise a metal (e.g., silver).

The biomaterial may be used in a wound dressing, e.g., in film form, in sponge form etc.

Also disclosed but not according to the invention is the biomaterial for use in a method for treating a wound in a subject in need thereof comprising administering an effective amount of the biomaterial described herein, e.g., in film form, in sponge form, etc., to a wound site present in the subject. The wound site may comprise a biofilm and administration of the biomaterial as described herein, e.g., in film form, in sponge form, etc., may prevent, reduce, inhibit, disrupt and/or remove the biofilm.

Also disclosed but not according to the invention is a method for preparing the biomaterial as described herein, e.g., in film form, in sponge form, etc. The method may comprise adding a solution comprising an antimicrobial agent (e.g., citric acid) to an intermediate slurry comprising collagen to form a biomaterial slurry. The antimicrobial agent (e.g., citric acid) may be added in an amount such that the film has a citric acid concentration > about 20 mM. The intermediate slurry comprises an anionic polysaccharide (e.g., oxidized regenerated cellulose (ORC)) and optionally a metal (e.g., silver). The method may also comprise drying or dehydrating the biomaterial slurry.

Also disclosed herein but not according to the invention is a method for preparing the biomaterial as described herein e.g., in film form, in sponge form, etc., comprising contacting the collagen with an acid solution, for example comprising (i) citric acid or (ii) citric acid and acetic acid to form a swelled collagen. The method further comprises combining the swelled collagen with an anionic polysaccharide (e.g., oxidized regenerated cellulose (ORC)) and optionally a metal (e.g., silver) to form a biomaterial slurry. The method may also comprise drying or dehydrating the biomaterial slurry.

The references to methods of treatment in paragraphs 54 and in the section "IV Wound therapy and Anti-Biofilm Uses" of this description are to be interpreted as references to the compounds, pharmaceutical compositions and medicaments of the present invention for use in a method for treatment of the human (or animal) body by therapy ."

Objectives, advantages, and illustrative modes of making and using the present technology may be understood by reference to the accompanying drawings in conjunction with the following detailed description of illustrative embodiments.

### DRAWINGS

FIG. 1 illustrates a simplified schematic diagram of an example embodiment of a negative pressure wound therapy system including a dressing. FIG. 1 is a perspective, cross-sectional view of a wound dressing according to the present technology.
FIG. 2 illustrates a colony drip-flow reactor (C-DFR) biofilm model used to grow *Pseudomonas aeruginosa* biofilms as described in Example 3.
FIG. 3 illustrates a log reduction of 72 hour old *Pseudomonas aeruginosa* biofilm total viable counts (TVC) compared to Tₒ for the following test samples: gauze, IODOFLEX, AQUACEL^{®} Ag+ EXTRA^{™}, collagen/ORC/silver-ORC, NEXT SCIENCE GEL, PRONTONSAN^{®}, Sponge Sample 2, Sponge Sample 3, and Sponge Sample 4.
FIG. 4 illustrates a reduction of 72 hour old *Pseudomonas aeruginosa* biofilm TVC for the following test samples: collagen/ORC/silver-ORC, Sponge Sample 4, Sponge Sample 3, Sponge Sample 2, Sponge Sample 1, and collagen/ORC/silver-ORC swelled with 200 mM acetic acid, and gauze.
FIG. 5 illustrates a reduction of 72 hour old *Pseudomonas aeruginosa* biofilm TVC for the following test samples: gauze, Sponge Sample 1, gauze + 100 mM citric acid, and collagen/ORC/silver-ORC.
FIG. 6 illustrates a reduction of 72 hour old *Pseudomonas aeruginosa* biofilm TVC for the following test samples: gauze, Sponge Sample 6, Sponge Sample 7, gauze + 100 mM citric acid, collagen/ORC, collagen/ORC/silver-ORC and Film Sample 8.

It should be noted that the figures set forth herein is intended to exemplify the general characteristics of materials and methods among those of the present technology, for the purpose of the description of certain embodiments. The figures may not precisely reflect the characteristics of any given embodiment, and are not necessarily intended to define or limit specific embodiments within the scope of this technology.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

While antimicrobial effects of acids (such as citric acid), and metals (such as silver) may be known, the biomaterials described herein unexpectedly exhibit synergistic effects in preventing, reducing, inhibiting, disrupting and/or removing a biofilm when compared to application of an antimicrobial agent such as citric acid alone, and as compared to application of a biomaterial comprising collagen, ORC, and an ORC-silver complex, such as PROMOGRAN PRISMA^{™} Matrix (available from Acelity) alone. In other words, the reduction in biofilm levels achieved by the biomaterials described herein is more than additive, *e.g.*, more than expected when compared to a reduction in the biofilm by an antimicrobial agent, such as citric acid, and a reduction in the biofilm by a biomaterial comprising collagen, ORC, and an ORC-silver complex.

Furthermore, the biomaterials described herein can prevent, reduce, inhibit, disrupt and/or remove a biofilm with little or no corresponding cytotoxicity to host cells, which otherwise can prevent and/or hinder wound healing. An antimicrobial agent *(e.g.,* citric acid) concentration has to be high enough to be toxic to bacterial cells but low enough so as to not be toxic to host cells, thereby creating a concentration window, if one exists. Achieving such a concentration window can be especially challenging and may not even exist when treating biofilms because some biofilms have an increased recalcitrance to antimicrobial agents, which may require high concentrations of antimicrobial agents *(e.g.,* citric acid) that may also be cytotoxic to host cells. However, it was unexpectedly discovered that higher concentrations of an antimicrobial agent *(e.g.,* citric acid) may be present in the biomaterial described herein without being substantially cytotoxic to host cells. Without wishing to be bound by theory, it is believed that higher concentrations of an antimicrobial agent *(e.g.,* citric acid) present in the biomaterial may be sufficient to disrupt the biofilm without being substantially cytotoxic to host cells wherein, for example, exposure of the antimicrobial agent (citric acid) to a biofilm occurs for a shorter duration. For example, wound healing may occur even when the biomaterial may be applied to the wound for a shorter amount of time. In some embodiments, the antimicrobial agent (citric acid) can be present in a concentration window, which may be high enough to disrupt the biofilm, but the exposure of the antimicrobial agent (citric acid) may be short enough to avoid cytotoxicity to host cells. For example, the antimicrobial agent (citric acid) may be present in a higher concentration, *e.g.*, ≥ about 200 mM, ≥ about 250 mM, ≥ about 300 mM, ≥ about 350 mM, ≥ about 400 mM, ≥ about 450 mM, ≥ about 500 mM, *etc.*

### I. Definitions

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

As used herein, the term "biomaterial" refers to a natural, synthetic, living, or non-living substance or material that may interact with biological systems and/or have a biological use. The term "biomaterial" is intended to encompass a material or substance that may have been engineered to take a form which, alone or as part of a complex system, may be used to direct, by control of interactions with components of living systems, the course of any therapeutic or diagnostic procedure. The term "biomaterial" is further intended to include a material that is biocompatible with a human or animal body. A biomaterial may comprise collagen.

As used herein, the term "biofilm" refers to an association of microorganisms, *e.g.,* single or multiple species, that can be encased or embedded in a matrix material, which may be self-produced by resident microorganisms. The biofilm may be present or adhere to living and/or non-living surfaces, *e.g*., tissue, a wound, medical implants, such as but not limited to orthopedic implants, dental implants, catheters, stents and so on. Exemplary microorganisms include, but are not limited to bacteria, *e.g*., Gram-negative bacteria, such as *Pseudomonas aeruginosa,* Gram-positive bacteria, such as *Staphylococcus aureus* and *Streptococcus mutans,* and non-bacterial microorganisms, such as yeasts, *e.g., Candida albicans.* The term "matrix material" is intended to encompass extracellular polymeric substances. Exemplary matrix materials include, but are not limited to polysaccharides, glycoproteins and/or nucleic acids. The term "biofilm" is further intended to include biological films that develop and persist at interfaces in aqueous environments. The language "biofilm development" or "biofilm formation" is intended to include the formation, growth, and modification of the bacterial colonies contained with biofilm structures, as well as the synthesis and maintenance of the exopolysaccharide matrix of the biofilm structures.

As used herein, the term "effective amount" refers to a quantity sufficient to achieve a desired therapeutic and/or prophylactic effect, *e.g.,* an amount which results in the prevention of, a decrease in or an amelioration of a condition described herein. In the context of therapeutic or prophylactic applications, the amount of a biomaterial administered to the subject will vary depending on the biomaterial, the degree, type, and severity of the wound and on the characteristics of the individual, such as general health, age, sex, body weight and tolerance to drugs. The skilled artisan will be able to determine appropriate amounts depending on these and other factors. The biomaterials can also be administered in combination with one or more additional therapeutic compounds. An effective amount can be given in one or more administrations.

As used herein, the terms "individual", "patient", or "subject" are used interchangeably and refer to an individual organism, a vertebrate, a mammal, or a human. In certain embodiments, the individual, patient or subject is a human.

As used herein, "prevention" or "preventing" of a condition (such as biofilm formation) refers to one or more compositions or biomaterials that, in a statistical sample, reduces the occurrence of the condition in the treated sample relative to an untreated control sample, or delays the onset of the condition relative to the untreated control sample.

As used herein, the term "tissue site" broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue.

"Treating", "treat", or "treatment" as used herein covers the treatment of a wound, in a subject, such as a human, and includes: (i) inhibiting or arresting development of a wound; (ii) relieving or causing regression of the wound; (iii) slowing progression of the wound; and/or (iv) inhibiting, relieving, or slowing progression of one or more symptoms of the wound.

It is also to be appreciated that the various modes of treatment or prevention of conditions as described herein are intended to mean "substantial," which includes total but also less than total treatment or prevention, and wherein some biologically or medically relevant result is achieved.

### II. Biomaterials

The biomaterials described herein are antimicrobial biomaterials and may exhibit anti-biofilm properties as discussed herein. The biomaterials of the present technology comprise collagen and an antimicrobial agent. Examples of suitable collagens include, but are not limited to native collagens, such as Types I, II and/or III native collagens, atelopeptide collagens, partially hydrolyzed collagens, such as gelatin, regenerated collagen and combinations thereof. The collagen may be present in any suitable amount, *e.g*., based on the total weight of the biomaterial. For example, collagen may be present in an amount ≥ about 25 wt%, ≥ about 30 wt%, ≥ about 35 wt%, ≥ about 40 wt%, ≥ about 45 wt%, ≥ about 50 wt%, ≥ about 55 wt%, ≥ about 60 wt%, ≥ about 65 wt%, ≥ about 70 wt%, ≥ about 75 wt%, or ≥ about 80 wt%. Additionally or alternatively, in some embodiments, collagen may be present in an amount of about 25 wt% to about 80 wt%, about 35 wt% to about 75 wt%, about 40 wt% to about 70 wt%, about 45 wt% to about 65 wt%, or about 50 wt% to about 60 wt% based on the total weight of the biomaterial.

Examples of further suitable antimicrobial agents present in the biomaterial of the present technology include, but are not limited to, organic acids such as carboxylic acids, silver, gold, zinc, copper, polyhexamethylene biguanide (PHMB), iodine and combinations thereof. Exemplary carboxylic acids include, but are not limited to ascorbic acid (*e.g.*, (R)-3,4-dihydroxy-5-((S)- 1,2-dihydroxyethyl)furan-2(5H)-one or Vitamin C), formic acid, gluconic acid, lactic acid, oxalic acid, tartaric acid, peroxy-pyruvic acid, and combinations thereof. Other examples of carboxylic acids include acetic acid (*i.e.*, ethanoic acid). Citric acid is present in the biomaterial of the present technology. Citric acid may be present in the biomaterial of the present technology in a suitable concentration, *e.g*., a concentration sufficient to reduce bacteria concentration in a wound, including reducing bacterial biofilms, in order to promote wound healing and/or control infection. Without wishing to be bound by theory, it is believed that citric acid can disrupt a biofilm, for example, by disrupting the extracellular matrix and exposing the bacteria to the biomaterial and thus, positively affecting and promoting wound healing.

In various aspects, citric acid may be present in a concentration ≥ about 15 mM, ≥ about 20 mM, ≥ about 25 mM, ≥ about 50 mM, ≥ about 75 mM, ≥ about 100 mM, ≥ about 125 mM, ≥ about 150 mM, ≥ about 175 mM, ≥ about 200 mM, ≥ about 225 mM, ≥ about 250 mM, ≥ about 275 mM, ≥ about 300 mM, ≥ about 325 mM, ≥ about 350 mM, ≥ about 375 mM, ≥ about 400 mM, ≥ about 425 mM, ≥ about 450 mM, ≥ about 475 mM, ≥ about 500 mM, ≥ about 525 mM, ≥ about 550 mM, ≥ about 575 mM, ≥ about 600 mM, ≥ about 625 mM, or ≥ about 650 mM. In some embodiments, citric acid may be present in a concentration ≥ about 20 mM. Additionally or alternatively, in some embodiments, citric acid may be present in a concentration of about 15 mM to about 650 mM, about 20 mM to about 500 mM, about 20 mM to about 400 mM, about 50 mM to about 650 mM, about 50 mM to about 500 mM, about 50 mM to about 400 mM, about 75 mM to about 650 mM, about 75 mM to about 500 mM, about 75 mM to about 400 mM, about 100 mM to about 650 mM, about 100 mM to about 500 mM, or about 100 mM to about 400 mM.

The biomaterial comprises an anionic polysaccharide, oxidized cellulose.

The oxidized cellulose present in the biomaterial of the present technology is oxidized regenerated cellulose (ORC), which may be prepared by oxidation of a regenerated cellulose, such as rayon. It has been known that ORC has hemostatic properties. ORC has been available as a hemostatic fabric called SURGICEL^{®} (Johnson & Johnson Medical, Inc.) since 1950. This product may be produced by the oxidation of a knitted rayon material.

ORC may be present in the biomaterial in any suitable amount, *e.g.*, based on the total weight of the biomaterial. ORC may be present in an amount ≥ about 15 wt%, ≥ about 20 wt%, ≥ about 25 wt%, ≥ about 30 wt%, ≥ about 35 wt%, ≥ about 40 wt%, ≥ about 45 wt%, ≥ about 50 wt%, ≥ about 55 wt%, ≥ about 60 wt%, ≥ about 65 wt%, or ≥ about 70 wt% based on the total weight of the biomaterial. Additionally or alternatively, in some embodiments, ORC may be present in the biomaterial of the present technology in an amount of about 15 wt% to about 70 wt%, about 20 wt% to about 65 wt%, about 25 wt% to about 65 wt%, about 30 wt% to about 60 wt%, about 35 wt% to about 55 wt%, or about 40 wt% to about 50 wt% based on the total weight of the biomaterial.

A biomaterial comprising collagen, citric acid and ORC are provided herein. For example, the biomaterial may comprise PROMOGRAN^{™} Matrix (available from Acelity) and citric acid.

In various embodiments, the biomaterial may further comprise a metal, for example silver, which may be used as a further antimicrobial agent. The metal (*e.g*., silver) may be present in metallic form, in ionic form *(e.g.,* a silver salt), or both. In some embodiments, silver may be present in combination with one or more additional metals, for example, gold, platinum, ferro-manganese, copper, zinc, or combinations thereof. The metal, particularly, silver, may confer antimicrobial properties to the biomaterial and in sufficiently lower concentrations, *e.g*., about 0.10 wt% to about 3.0 wt%, the silver may not cause cytotoxicity in a wound or at a tissue site.

In some embodiments, at least a portion of the metal may be present as an ORC-silver complex. As used herein, the term "complex" refers to an intimate mixture at the molecular scale, suitably with ionic or covalent bonding between the metal (*e.g.*, silver) and ORC. The complex may comprise a salt formed between the anionic polysaccharide and Ag⁺, but it may also comprise silver clusters and/or colloidal silver metal, for example produced by exposure of the complex to light. For example, ORC may be treated with a silver salt solution to produce a complex of ORC with silver. The silver salt solution may be an aqueous solution and the solution may be prepared in a quantity sufficient to provide the desired silver concentration in the resultant complex. In some embodiments, the amount of silver in the complex may be from about 0.1% to about 50% by weight based on the weight of ORC, particularly, from about 1% to about 40%, about 2% to about 30% by weight, or about 5% to about 25%.

In various embodiments, the ORC-silver complex may be present in the biomaterial of the present technology in an amount ≥ about 0.10 wt%, ≥ about 0.50 wt%, ≥ about 1.0 wt%, ≥ about 2.0 wt%, ≥ about 3.0 wt%, ≥ about 4.0 wt%, ≥ about 5.0 wt%, ≥ about 6.0 wt%, ≥ about 8.0 wt%, or ≥ about 10 wt%. Additionally or alternatively, the ORC-silver complex may be present in the biomaterial of the present technology in an amount of about 0.10 wt% to about 10 wt%, about 0.10 wt% to about 8.0 wt%, about 0.10 wt% to about 5.0 wt%, about 0.50 wt% to about 4.0 wt%, about 0.50 wt% to about 3.0 wt%, or about 0.50 wt% to about 2.0 wt% based on the total weight of the biomaterial.

A biomaterial comprising collagen, citric acid , ORC and a metal (*e.g.*, silver) are provided herein. For example, the biomaterial may comprise PROMOGRAN PRISMA^{™} Matrix (available from Acelity) and citric acid.

Advantageously, in addition to reducing vegetative or free-flowing bacteria, it was unexpectedly discovered that the biomaterials described may be capable of preventing, reducing, inhibiting, disrupting and/or removing a biofilm, *e.g.,* a biofilm present in a wound site, on tissue, on an implant, *etc.* In various aspects, the biomaterials described herein may be capable of a percentage reduction of a biofilm of about ≥ 10%, about ≥ 20%, about ≥ 30%, about ≥ 40%, about ≥ 50%, about ≥ 60%, about ≥ 70%, about ≥ 80%, about ≥ 90%, about ≥ 95%, or about ≥ 99%. Reducing a biofilm includes reducing the number of total viable microorganisms making up at least part of the biofilm, for example, as measured by total viable counts (TVC) of microorganisms (e.g., bacteria, yeast). The biofilm may comprise bacteria including, but not limited to *Pseudomonas aeruginosa, Staphylococcus aureus* and *Streptococcus mutans.* The biofilm may also include other non-bacterial microorganisms including but not limited to yeasts, such as *Candida albicans.* In some embodiments, the biomaterial described herein may be capable of reducing the biofilm, *e.g.*, after about 24 hours *in vitro* exposure, by about ≥ 1 log₁₀ units, about ≥ 2 log₁₀ units, about ≥ 3 log₁₀ units, about ≥ 4 log₁₀ units, about ≥ 5 log₁₀ units, or about ≥ 6 log₁₀ units, for example, wherein the biofilm comprises bacteria, such as *Pseudomonas aeruginosa.* Additionally or alternatively, in some embodiments, the biomaterial described herein may be capable of reducing the biofilm, *e.g.,* after about 24 hours *in vitro* exposure, by about 1 log₁₀ units to about 6 log₁₀ units, by about 2 log₁₀ units to about 6 log₁₀ units, by about 2 log₁₀ units to about 5 log₁₀ units or by about 3 log₁₀ units to about 5 log₁₀ units, for example, wherein the biofilm comprises bacteria, such as *Pseudomonas aeruginosa.*

In some embodiments, the biomaterials described herein can comprise openings defined therein of any suitable dimension and configuration. For example, the openings may by perforations, through-holes, channels, and the like. In some embodiments, these openings can be used as flow channels during wound therapy, such as negative pressure wound therapy, as further described below.

### A. Biomaterial Forms

The biomaterials described herein may be present in various forms. Suitable forms include, but are not limited to a sponge, a film, a foam, a gel, a bead, a rope, a polymeric matrix, a coating, a solution and combinations thereof. It is contemplated herein that in coating form, the biomaterial may be coated onto synthetic material, such as, but not limited to a mesh, a foam, or an implant. It is further contemplated herein that in solution form, the biomaterial may be utilized in instillation therapy.

In some embodiments, the biomaterials described herein are in the form of a sponge, *e.g.*, sponge is provided herein, which comprises collagen as described herein, citric acid and ORC, and optionally metal (*e.g.*, silver) as described herein, *e.g.*, an ORC-silver complex. In some embodiments, the sponge may comprise citric acid in a concentration ≥ about 20 mM, *e.g.*, about 20 mM to about 600 mM, about 20 mM to about 400 mM *etc.* In some embodiments, the sponge may comprise ORC in an amount of about 25 wt% to about 65 wt% or about 40 wt% to about 50 wt% based on the total weight of the sponge. In some embodiments, the sponge may comprise an ORC-silver complex in an amount of about 0.10 wt% to about 3.0 wt% or about 0.50 wt% to about 5.0 wt% based on the total weight of the sponge. In various embodiments, the sponge may be capable of preventing, reducing, inhibiting or removing a biofilm as described herein, e.g., present in or on a wound site, a tissue, an implant, *etc.*

In sponge form, the citric acid may be present within the collagen. Alternatively, in sponge form, citric acid may not be present within the collagen. In various aspects, the sponge may have an average pore size of about 10 µm to about 500 µm or about 100 µm to about 300 µm.

In some embodiments, the biomaterials described herein are in the form of a film, *i.e.*, a film is provided herein, which may comprise collagen as described herein, citric acid, ORC, and optionally metal (*e.g.*, silver) as described herein, *e.g.*, an ORC-silver complex. In some embodiments, the film may comprise citric acid in a concentration ≥ about 20 mM, *e.g.*, about 20 mM to about 600 mM, about 20 mM to about 400 mM *etc.* In some embodiments, the film may comprise ORC in an amount of about 25 wt% to about 65 wt% or about 40 wt% to about 50 wt% based on the total weight of the film. In some embodiments, the film may comprise ORC-silver complex in an amount of about 0.10 wt% to about 3.0 wt% or about 0.50 wt% to about 5.0 wt% based on the total weight of the film. In various embodiments, the film may be capable of preventing, reducing, inhibiting, disrupting or removing a biofilm as described herein, e.g., present in or on a wound site, a tissue, an implant, *etc.*

In some embodiments, the film may be flexible or rigid. In some embodiments, the film may further comprise a plasticizer, such as glycerol, in a suitable amount, *e.g.,* to render the film more flexible. In various aspects, the film may be continuous or interrupted (e.g., perforated).

In some embodiments, the film may be substantially transparent. In some embodiments, the film may further comprise a grid of any suitable dimension, for example, 0.50 cm by 0.50 cm, 1.0 cm by 1.0 cm, 1.5 cm by 1.5 cm, 2.0 cm by 2.0 cm, *etc.*

In some embodiments, the biomaterials described herein are present in a multilayer configuration. For example, biomaterials described herein may comprise at least two layers, *e.g.,* a first layer, a second layer, a third layer, *etc.,* wherein the first layer contacts the wound site, *i.e.,* maybe considered a "wound interface layer." A first layer may comprise one or more antimicrobial agents (*e.g.*, citric acid, silver, PHMB) as described herein. In some embodiments, the antimicrobial agent (*e.g.*, citric acid, silver, PHMB) present in the first layer may be present in a higher concentration, *e.g.*, ≥ about 200 mM, ≥ about 250 mM, ≥ about 300 mM, ≥ about 350 mM, ≥ about 400 mM, ≥ about 450 mM, ≥ about 500 mM, *etc.* In some embodiments, the first layer comprises citric acid. In some embodiments, the first layer comprises silver and/or PHMB. In some embodiments, the first layer may further comprise ORC as described herein and collagen as described herein. A second layer may comprise ORC as described herein and collagen as described herein. In some embodiments, the second layer may further comprise the metal (*e.g.*, silver) as described herein, *e.g.*, an ORC-silver complex. In some embodiments, the first layer may be adjacent to the second layer. In other embodiments, the first layer and the second layer may be separated by one or more additional layers.

In some embodiments, a third layer may be present. The third layer may comprise collagen as described herein and ORC. The third layer may further comprise growth factors, for example, for supporting wound healing. Examples of growth factors include, but are not limited to, fibroblast growth factor, platelet derived growth factor, epidermal growth factor and combinations thereof.

### III. Antimicrobial Wound Dressings

Wound dressings comprising a biomaterial as described herein are also disclosed but are not part of the invention. Therefore, and as more fully described herein, a method of wound therapy is provided comprising administering a wound dressing comprising a biomaterial as described herein to a wound site present in a subject in need thereof. The wound dressings may be used for the treatment of wounds, especially chronic wounds such as venous ulcers, decubitis ulcers or diabetic ulcers. The biomaterial in/on the wound dressing may act as an antimicrobial agent to reduce, prevent, and/or disrupt a biofilm present in the wound. The wound dressing may be resorbable or non-resorbable.

In some embodiments, the wound dressing may be in the form of a sheet, for example a sheet of substantially uniform thickness. The area of the sheet typically may be from about 1 cm² to about 400 cm², and the thickness typically from about 1 mm to about 10 mm. The sheet may, for example, be a freeze-dried sponge, a film, or a knitted, woven or nonwoven fibrous sheet or a gel sheet. The sheet may comprise less than about 15% by weight of water, or less than about 10% by weight of water.

In various embodiments, the wound dressing may comprise an active layer of the biomaterial as described herein. The active layer contributes to preventing, reducing, inhibiting, disrupting or removing a biofilm. The active layer can be a wound interface layer in use, or alternatively, the active layer may be separated from the wound by a liquid-permeable top sheet. The area of the active layer may be from about 1 cm² to about 400 cm² or from about 4 cm² to about 100 cm². In some embodiments, the active layer contains one or more antimicrobial agents (*e.g.*, citric acid, silver and/or PHMB) of the biomaterial.

In some embodiments, the wound dressing may further comprise a backing sheet extending over the active layer opposite to the wound facing side of the active layer. The backing sheet may be larger than the active layer such that a marginal region of width 1 mm to 50 mm, or 5 mm to 20 mm extends around the active layer to form a so-called island dressing. In such cases, the backing sheet may be coated with a pressure sensitive medical grade adhesive in at least its marginal region.

In some embodiments, the backing sheet may be substantially liquid-impermeable. In particular, the backing sheet may be semipermeable. That is to say, the backing sheet may be permeable to water vapor, but not permeable to liquids (e.g., water) or wound exudate. Additionally or alternatively, in some embodiments, the backing sheet may also be microorganism-impermeable. Suitable continuous conformable backing sheets may have a moisture vapor transmission rate (MVTR) of the backing sheet alone of 300 to 5000 g/m²/24 hrs, or 500 to 2000 g/m²/24 hrs at 37.5°C at 100% to 10% relative humidity difference. The backing sheet thickness may be in a range of 10 to 1000 micrometers or 100 to 500 micrometers.

In some embodiments, the MVTR of the wound dressing as a whole may be lower than that of the backing sheet alone, because an apertured sheet can partially obstruct moisture transfer through the dressing. The MVTR of the dressing (measured across the island portion of the dressing) may be from 20% to 80% of the MVTR of the backing sheet alone, or from 20% to 60% thereof, or about 40% thereof. It has been found that such moisture vapor transmission rates can allow the wound under the dressing to heal under moist conditions without causing the skin surrounding the wound to macerate.

Suitable polymers for forming the backing sheet include, but are not limited to, polyurethanes and poly alkoxyalkyl acrylates and methacrylates such as those disclosed in GB-A-1280631. The backing sheet may comprise a continuous layer of a high density blocked polyurethane foam that may be predominantly closed-cell. A suitable backing sheet material is the polyurethane film available under the registered trademark ESTANE 5714F.

An adhesive layer (where present) can be moisture vapor transmitting and/or patterned to allow passage of water vapor through. The adhesive layer can be a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer may be 20 to 250 g/m², or 50 to 150 g/m². Polyurethane-based pressure sensitive adhesives may be used.

In some embodiments, the wound facing surface of the dressing may be protected by a removable cover sheet. The cover sheet may be formed from a flexible thermoplastic material. Suitable materials include, but are not limited to polyesters and polyolefins. Additionally or alternatively, in some embodiments, the adhesive-facing surface of a cover sheet may be a release surface. That is to say, a surface that may be only weakly adherent to a wound facing surface of the dressing and the adhesive on the backing sheet to assist peeling from the cover sheet. For example, the cover sheet may be formed from a non-adherent plastic such as a fluoropolymer, or it may be provided with a release coating such as a silicone or fluoropolymer release coating. In some embodiments, further layers of a multilayer absorbent article may be built up between the active layer and a protective sheet, e.g., the backing sheet and/or the removable cover sheet. For example, these layers may comprise an apertured plastic film to provide support for the active layer in use.

In some embodiments, the dressing may further comprise an absorbent layer between the active layer and the protective removable cover sheet, particularly if the dressing may be for use on exuding wounds. The optional absorbent layer may be any of the layers conventionally used for absorbing wound fluids, serum or blood in the wound healing art, including gauzes, nonwoven fabrics, superabsorbents, hydrogels and mixtures thereof. The absorbent layer may comprise a layer of absorbent foam, such as an open celled hydrophilic polyurethane foam prepared in accordance with EP-A-0541391. In other embodiments, the absorbent layer may be a nonwoven fibrous web, for example a carded web of viscose staple fibers. The basis weight of the absorbent layer may be in the range of 50-500 g/m², such as 100-400 g/m². The uncompressed thickness of the absorbent layer may be in the range of from 0.5 mm to 10 mm, such as 1 mm to 4 mm. The free (uncompressed) liquid absorbency measured for physiological saline may be in the range of 5 to 30 g/g at 250. The absorbent layer or layers may be substantially coextensive with the biomaterial comprising the OCR-silver complex.

Additionally, the wound dressings and materials may be sterilized, for example, by gamma-irradiation. In some embodiments, the sterility assurance level is better than 10⁻⁶. The wound dressings may be packaged in a microorganism-impermeable container.

### IV. Wound Therapy and Anti-Biofilm Uses

The biomaterials as described herein have anti-biofilm properties, such that the biomaterials can reduce biofilm total viable counts (TVC) and/or prevent biofilm growth. Therefore, methods for preventing, reducing, inhibiting, disrupting and/or removing a biofilm as described herein are provided. The methods may comprise contacting the biofilm or contacting a cell capable of forming a biofilm with a biomaterial described herein, *e.g.,* a biomaterial film, a wound dressing comprising the biomaterial, *etc.*

The biomaterials described herein may reduce the biofilm by about ≥ 10%, about ≥ 20%, about ≥ 30%, about ≥ 40%, about ≥ 50%, about ≥ 60%, about ≥ 70%, about ≥ 80%, about ≥ 90%, about ≥ 95%, or about ≥ 99%. For example, during the methods described herein, the biomaterial described herein may reduce the biofilm, *e.g.*, after about 24 hours *in vitro* exposure, by about ≥ 1 log₁₀ units, about ≥ 2 log₁₀ units, about ≥ 3 log₁₀ units, about ≥ 4 log₁₀ units, about ≥ 5 log₁₀ units, or about ≥ 6 log₁₀ units, for example, wherein the biofilm comprises bacteria, such as *Pseudomonas aeruginosa.* Additionally or alternatively, in some embodiments of the methods of the present technology the biomaterial described herein may reduce the biofilm, *e.g.,* after about 24 hours *in vitro* exposure, about 1 log₁₀ units to about 6 log₁₀ units, about 2 log₁₀ units to about 6 log₁₀ units, about 2 log₁₀ units to about 5 log₁₀ units or about 3 log₁₀ units to about 5 log₁₀ units, for example, wherein the biofilm comprises bacteria, such as *Pseudomonas aeruginosa.*

In additional embodiments, the biomaterials described herein can be used in wound therapy or healing. The methods may comprise applying a biomaterial as described herein, *e.g.,* a biomaterial film, a wound dressing comprising the biomaterial, *etc.,* to a wound site. In various embodiments, the wound site may comprise a biofilm as described herein and the biomaterial may prevent, reduce, disrupt or inhibit growth of the biofilm, or remove the biofilm.

Additionally or alternatively, in some embodiments, the biomaterial described herein may be employed in therapy in which a tissue site, for example, a wound, may be treated with reduced pressure. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure."

"Negative pressure" may generally refer to a pressure less than a local ambient pressure, such as the ambient pressure in a local environment external to a sealed therapeutic environment provided by a dressing. In many cases, the local ambient pressure may also be the atmospheric pressure proximate to or about a tissue site. Alternatively, the pressure may be less than a hydrostatic pressure associated with the tissue at the tissue site. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa), gauge pressure. Common therapeutic ranges are between -50 mm Hg (-6.7 kPa) and -300 mm Hg (-39.9 kPa), gauge pressure.

Negative-pressure therapy may provide a number of benefits, including migration of epithelial and subcutaneous tissues, improved blood flow, and micro-deformation of tissue at a wound site. Together, these benefits may increase development of granulation tissue and reduce healing times.

In various aspects, a negative-pressure wound therapy may comprise positioning the biomaterial proximate to a tissue site, such as a wound. The negative-pressure therapy may further comprise sealing the biomaterial to tissue surrounding the tissue site or wound site to form a sealed space. For example, a cover may be placed over the biomaterial and sealed to an attachment surface near the tissue site, such as undamaged epidermis peripheral to a tissue site.

The negative-pressure therapy method may further comprise fluidly coupling a negative-pressure source to the sealed space and operating the negative-pressure source to generate a negative pressure in the sealed space. For example, the negative-pressure source may be coupled to the biomaterial such that the negative-pressure source may be used to reduce the pressure in the sealed space. In some embodiments, negative pressure applied across a tissue site, for example, via the biomaterial may be effective to induce macrostrain and microstrain at the tissue site or wound site, as well as remove exudates and other fluids from the tissue site.

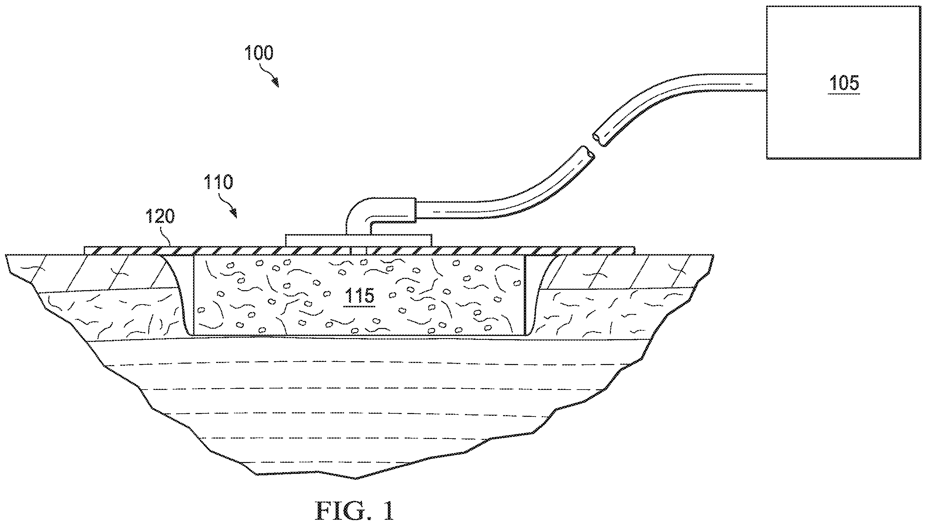

FIG. 1 is a simplified schematic that illustrates an example embodiment of a system 100 that can provide negative-pressure therapy. Generally, the system 100 may be configured to provide negative-pressure to a tissue site. In various embodiments, the system 100 generally includes a negative-pressure supply, such as a negative-pressure source 105, and may include or be configured to be coupled to a distribution component. In general, a distribution component may refer to any complementary or ancillary component configured to be fluidly coupled to a negative-pressure supply in a fluid path between a negative-pressure supply and a tissue site. For example, in the embodiment of FIG. 1, a dressing 110 is an example of a distribution component that is fluidly coupled to the negative-pressure source 105. As illustrated in the example of FIG. 1, the dressing 110 may comprise or consist essentially of a tissue interface 115, a cover 120, or both in some embodiments. In some embodiments, the tissue interface 115 may be in the form of a film or sponge comprising the biomaterial as described herein, optionally further comprising additional manifold material, for example as a single layer. In some embodiments, the dressing 110 may be multi-layered. For example, the tissue interface 115 in the form of a film or sponge comprising the biomaterial as described herein may be considered a first layer, and a second layer comprising foam may be adjacent to the first layer.

Some components of the system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with a controller and other components into a therapy unit.

In general, components of the system 100 may be coupled directly or indirectly. Coupling may include fluid, mechanical, thermal, electrical, or chemical coupling (such as a chemical bond), or some combination of coupling in some contexts. In some embodiments, components may also be coupled by virtue of physical proximity, being integral to a single structure, or being formed from the same piece of material.

In various embodiments, components may be fluidly coupled to each other to provide a path for transferring fluids between the components. For example, components may be fluidly coupled through a fluid conductor. A "fluid conductor," in this context, broadly includes a tube, pipe, hose, conduit, or other structure with one or more lumina or passageways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from KCI of San Antonio, Texas.

In various embodiments, a negative-pressure supply, such as the negative-pressure source 105, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example.

The tissue interface 115 can be generally adapted to contact a tissue site. The tissue interface 115 may be partially or fully in contact with a tissue site. If the tissue site is a wound, for example, the tissue interface 115 may partially or completely fill the wound, or may be placed over the wound. The tissue interface 115 may take many forms, and may have many sizes, shapes, or thicknesses depending on a variety of factors, such as the type of treatment being implemented or the nature and size of a tissue site. For example, the size and shape of the tissue interface 115 may be adapted to the contours of deep and irregular shaped tissue sites. Moreover, any or all of the surfaces of the tissue interface 115 may have projections or an uneven, course, or jagged profile that can induce strains and stresses on a tissue site, which can promote granulation at the tissue site.

The tissue interface 115 may also be generally configured to distribute negative pressure so as to collect fluid. In some embodiments, for example, the tissue interface 115 may comprise or be configured as a manifold. A "manifold" in this context generally includes any composition or structure providing a plurality of pathways configured to collect or distribute fluid across a tissue site under pressure. For example, the tissue interface 115 may be in the form of a film or a sponge comprising the biomaterial described herein, and may include openings or punctures, *e.g.,* perforations, through-holes, *etc.,* to allow for it to manifold fluid and/or pressure.

In some embodiments, the fluid pathways of a manifold may be interconnected to improve distribution or collection of fluids. In some embodiments, a manifold may be a porous material having a plurality of interconnected cells or pores. For example, open-cell foam, gauze, or felted mat material generally includes pores, edges, or channels that are interconnected, and may be suitable for use as a manifold material. The average pore size may vary according to needs of a prescribed therapy. For example, in some embodiments, the tissue interface 114 may be reticulated foam having pore sizes in a range of 400-600 microns. The tensile strength of the tissue interface 114 may also vary according to needs of a prescribed therapy. In one non-limiting example, the tissue interface 114 may comprise reticulated polyurethane foam such as used in GRANUFOAM^{™} dressing available from Acelity of San Antonio, Texas.

For example, a manifold may be configured to receive negative pressure from the negative-pressure source 105 and to distribute negative pressure through multiple apertures (e.g., pores), which may have the effect of collecting fluid and drawing the fluid toward the negative-pressure source 105. More particularly, in the embodiment of FIG. 1, the dressing 110 may be configured to receive negative pressure from the negative-pressure source 105 and to distribute the negative pressure through the tissue interface 115, for example, which may have the effect of collecting fluid from the tissue site through the tissue interface 115. In additional or alternative embodiments, the fluid path may be reversed or a secondary fluid path may be provided to facilitate movement of fluid across a tissue site.

In some embodiments, the cover 120 may provide a bacterial barrier and protection from physical trauma. The cover 120 may also be constructed from a material that can reduce evaporative losses and provide a fluid seal between two components or two environments, such as between a therapeutic environment and a local external environment. The cover 120 may be, for example, an elastomeric film or membrane that can provide a seal adequate to maintain a negative pressure at a tissue site for a given negative-pressure source. The cover 120 may have a high moisture-vapor transmission rate in some applications. For example, in some embodiments, the MVTR may be at least 300 g/m² per twenty-four hours. In some example embodiments, the cover 120 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained.

The fluid mechanics associated with using a negative-pressure source to reduce pressure in another component or location, such as within a sealed therapeutic environment, can be mathematically complex. However, the basic principles of fluid mechanics applicable to negative-pressure therapy are generally well-known to those skilled in the art. The process of reducing pressure may be described generally and illustratively herein as "delivering," "distributing," or "generating" negative pressure, for example.

In operation, the tissue interface 115 may be placed within, over, on, or otherwise proximate to a tissue site. The cover 120 may be placed over the tissue interface 115 and sealed to an attachment surface near the tissue site. For example, the cover 120 may be sealed to undamaged epidermis peripheral to a tissue site. Thus, the dressing 110 can provide a sealed therapeutic environment proximate to a tissue site, substantially isolated from the external environment, and the negative-pressure source 105 can reduce the pressure in the sealed therapeutic environment. Negative pressure applied across the tissue site through the tissue interface 115 in the sealed therapeutic environment can remove exudates and other fluids from the tissue site. Additionally, such configurations may allow for therapeutic levels of negative pressure to be achieved, while providing an environment conducive for granulation and cellular regeneration at the wound interface. Further, tissue ingrowth, for example into the tissue interface 115, may be prevented, which can damage newly formed tissue upon removal and/or changing of the dressing 100.

### V. Methods of Preparing the Biomaterials

Methods of preparing the biomaterials as described herein are also disclosed ( not part of the invention). The method may comprise adding a solution comprising citric acid to an intermediate slurry comprising collagen as described herein to form a biomaterial slurry. The solution comprising citric acid may be prepared by mixing a suitable amount of citric acid, for example, in powdered form or liquid form, with a solvent, such as water, to form the solution comprising citric acid in a concentration such that the resultant biomaterial, after mixing with the intermediate slurry, has a citric acid concentration as described herein, *e.g.*, ≥ about 20 mM, ≥ about 50 mM, ≥ about 100 mM, or about 20 mM to about 600 mM, about 20 mM to about 400 mM, *etc.*

The intermediate slurry comprises ORC in a suitable amount as described herein. Additionally, the intermediate slurry may further comprise a metal (*e.g.*, silver) as described herein in a suitable amount as described herein. As discussed above, at least a portion of the metal (*e.g.*, silver) as described herein may be present as an ORC-silver complex. In some embodiments, this complex may be prepared by treating ORC with a solution of a metal salt (*e.g.*, silver salt). The complex may comprise a salt formed between ORC and the metal ion (*e.g.*, Ag⁺). The metal salt solution may be an aqueous solution, and can be prepared in a quantity sufficient to provide the desired metal (e.g., silver) concentration as described herein in the resulting complex.

Anionic polysaccharides may behave as an ion exchanger and can pull out of solution a metal ion (*e.g.*, Ag⁺) of a metal salt (*e.g.*, silver salt) that contacts the anionic polysaccharides. The by-product of this exchange may be an acid from the salt and by using a salt of a weak organic acid, a weak acid may be produced which may not damage the polysaccharide. Using salts of strong acids such as sodium chloride or sodium sulfate produces hydrochloric acid or sulfuric acid by-products respectively, and these strong acids can cause damage such as depolymerization of the polysaccharide.

When using metal salts *(e.g.,* silver salts) of weak acids, the metal ion *(e.g.,* silver ion) may be exchanged for a proton on the polysaccharide and part of the salt is converted to weak acid. The mixture of acid and salt in the solution can result in a buffered solution which can maintain a fairly constant pH and can control the degree of neutralization. An equilibrium reaction may be established whereby the metal ions (e.g., silver ions) are bound to the acid portion of the polysaccharide and also to the salt molecules. This partitioning of the metal ions (e.g., silver ions) can prevent the neutralization of the polysaccharide from going to completion. Using a stoichiometric amount of, for example, silver acetate brings about a 65-75% degree of neutralization of the carboxylic acid groups on an oxidized cellulose polymer. This control of pH by creating a self-generating buffered solution and the use of methanol to control the swelling of the material can lead to a partially neutralized material in which the physical properties, e.g., tensile strength and shape of the polysaccharide, are preserved.

The amount of metal salt *(e.g.,* silver salt) used generally may be about equal to or up to twice the stoichiometric amount of carboxylic acid content of the polysaccharide. Alternatively, a second charge of a stoichiometric amount of metal salt *(e.g.,* silver salt) can be used if the reaction is recharged with fresh solvent and salt after the first charge reaches a constant pH. The material with elevated pH may then be washed to remove the excess metal salt *(e.g.,* silver salt) and ions therefrom.

The length of time that ORC may be treated with the metal salt solution is a period sufficient to incorporate the desired concentration of metal (*e.g.*, silver) into the complex. For example, ORC may be treated with the metal salt solution for between 1 and 120 minutes. In some embodiments, the treatment time may be about 10, 20, 30, 40, 50, 60 or more minutes.

In some embodiments, the ORC-silver complex may be mixed with a further anionic polysaccharide as described herein, *e.g.,* anionic polysaccharides that have not been complexed with a metal, as well as collagen to form the intermediate slurry. In particular, the further anionic polysaccharide may be ORC.

In some embodiments, the collagen may be contacted with an acid solution, *e.g.,* in order to swell the collagen. Examples of suitable acid solutions include, but are not limited to acetic acid and/or ascorbic acid. For example, the collagen may be contacted with the acid solution prior to forming the intermediate slurry with the ORC-silver complex and optionally, the further anionic polysaccharide (*e.g.*, ORC) and/or prior to adding the solution comprising the citric acid to the intermediate slurry.

In some embodiments, the method may further comprise adding a plasticizer, such as, but not limited to glycerol, in a suitable amount. For example, the plasticizer may be added to the intermediate slurry and/or to the biomaterial slurry.

In alternative embodiments, the methods may comprise contacting the collagen with an acid solution comprising (i) citric acid or (ii) citric acid and acetic acid in suitable amounts to form a swelled collagen. The swelled collagen may then be combined with ORC and a metal (*e.g.*, silver) in suitable amounts to form the biomaterial slurry. As discussed above, at least a portion of the metal (*e.g.*, silver) as described herein may be present as an ORC-silver complex. An ORC-silver complex may be prepared as discussed above. In some embodiments, the swelled collagen may then be combined with an ORC-silver complex and optionally, a further anionic polysaccharide (*e.g.*, ORC) as described herein in suitable amounts to form the biomaterial slurry. In some embodiments, the method may further comprise adding a plasticizer, such as, but not limited to glycerol, in a suitable amount. For example, the plasticizer may be combined with the swelled collagen, ORC and/or the metal (*e.g.*, silver).

In various embodiments, the methods described herein may further comprise drying or dehydrating the biomaterial slurry, *e.g.,* to form a sponge or a film. Drying may comprise freeze-drying or solvent-drying of the biomaterial slurry. Freeze-drying may comprise the steps of freezing the biomaterial slurry, followed by evaporating the solvent from the frozen biomaterial slurry under reduced pressure. Suitably, a method of freeze-drying is similar to that described for a collagen-based sponge in U.S. Pat. No. 2,157,224. In some embodiments, the freeze-drying may be performed in stages to prepare the multi-layered configurations described herein. In some embodiments, a first layer comprising biomaterial as described herein may be frozen at a suitable temperature until solid, for example about -80°C. A second layer comprising biomaterial as described herein may be added adjacent to the first layer by repeating the process until a desired composition is achieved. The resultant multi-layered configuration may be freeze-dried as described above.

Solvent-drying may comprise freezing the biomaterial slurry, followed by immersing the biomaterial slurry in a series of baths of a hygroscopic organic solvent such as anhydrous isopropanol to extract the water from the frozen biomaterial slurry, followed by removing the organic solvent by evaporation. Methods of solvent drying are described, for example, in U.S. Pat. No. 3,157,524.

In some embodiments, to form a biomaterial film as described herein, the biomaterial slurry as prepared as described above, may be placed in a dehydration oven, which may evaporate water and/or solvent using suitably higher temperatures with or without circulation of air through a chamber containing a desiccant or the like.

In some embodiments, the methods may further comprise treating the biomaterial slurry, or the dried biomaterial, with a cross-linking agent such as epichlorhydrin, carbodiimide, hexamethylene diisocyanate (HMDI) orglutaraldehyde . Alternatively, cross-linking may be carried out dehydrothermally. The method of cross-linking can affect the final product. For example, HMDI cross-links the primary amino groups on collagen, whereas carbodiimide cross-links carbohydrate on the ORC to primary amino groups on the collagen.

### VI. Advantages

The biomaterials and related uses described herein may provide significant advantages, for example, when used in wound therapy or with implants. As discussed herein, conventional attempts to control biofilms, for example, during wound healing, may be made difficult by the production of an extracellular matrix, which can anchor the biofilm to various living and non-living surfaces and/or may physically protect the bacterial cells within the extracellular matrix. In some embodiments, the biomaterial described herein may be effective to prevent, inhibit, reduce, and/or remove a biofilm, for example, by disrupting or degrading the extracellular matrix. Without wishing to be bound by theory, it is believed that the biomaterial may be effective to lower the pH in the proximity of the biofilm and disrupt the extracellular matrix, thereby exposing the bacteria within the extracellular matrix and rendering those bacteria susceptible to the antimicrobial activity of the biomaterial. For example, and not intending to be bound by theory, by disrupting the extracellular matrix, the biomaterial as described herein may have improved antimicrobial activity in comparison to a biomaterial that does not include an antimicrobial agent (such as citric acid), or in comparison to using an antimicrobial agent (such as citric acid) alone. Indeed, the biomaterials described herein exhibit synergistic effects in preventing, reducing, inhibiting and/or removing a biofilm when compared to application of an antimicrobial agent, such as citric acid, alone, and application of a biomaterial comprising collagen, ORC, and an ORC-silver complex, such as PROMOGRAN PRISMA^{™} Matrix (available from Acelity), without an antimicrobial agent, such as citric acid. Furthermore, the biomaterials described herein can prevent, reduce, inhibit, disrupt and/or remove a biofilm with little or no corresponding cytotoxicity to host cells, for example *in vitro,* which otherwise can prevent wound healing. For example, the antimicrobial agent *(e.g.,* citric acid) can be present in a concentration window, which may high enough to disrupt the biofilm, but the exposure of the antimicrobial agent (e.g., citric acid) may be short enough to avoid cytotoxicity to host cells.

### EXAMPLES

The benefits associated with the biomaterial and methods are further demonstrated by the following, non-limiting Examples. These Examples may demonstrate one or more features associated with some embodiments of the biomaterials and methods.

### Example 1-Preparation Method I of Biomaterial Sponge with Collagen/ORC/Silver-ORC and Citric Acid

An intermediate slurry comprising 55% collagen (1.1 g), 45% ORC (0.88 g) and 1% silver-ORC (0.02g) complex was prepared according to U.S. Patent No. 8,461,410. Citric acid in the amounts listed in Table 1 was solubilized in 5 ml of water and added to 80 ml of the intermediate slurry to prepare biomaterial slurries having varying citric acid concentrations. A portion (31 grams) of each of the biomaterial slurries with varying citric acid concentrations were transferred into 10 × 10 cm square plates and spread evenly before freezing at -80°C overnight and followed by freeze drying for 24 hours to prepare sponge Samples 1, 2, 3 and 4 having a citric acid concentration of 100 mM, 150 mM, 200 mM and 400 mM, respectively. Samples 1-4 were gamma sterilized before microbiological evaluation.

**Table 1**

| **Sponge Samples** | **Final Citric Acid Concentration (mM)** | **Citric Acid Amount Added** | |
|---|---|---|---|
| | | **(g/L)** | **(g/80ml)** |
| 1 | 100 | 19.21 | 1.536 |
| 2 | 150 | 28.82 | 2.305 |
| 3 | 200 | 38.42 | 3.072 |
| 4 | 400 | 76.84 | 6.144 |

### Example 2-Preparation Method II of Biomaterial Sponge with Collagen/ORC/Silver-ORC and Citric Acid

Collagen powder was added to an appropriate concentration of citric acid or mixture of both citric acid and acetic acid and mixed in a blender to form a mixture with 2% solid content (1.1 g per 100 ml). ORC (0.88 g per 100 ml) and silver-ORC (0.02g per 100 ml) were added to the mixture and blended to form a slurry. A portion (31 grams) of the slurry was transferred into 10 × 10 cm square plates and spread evenly before freezing at - 80°C overnight and followed by freeze drying for 24 hours to prepare a sponge Sample 5. Sample 5 was gamma sterilized before microbiological evaluation.

### Example 3-Preparation Method of Biomaterial Sponge with Collagen/ORC and Citric Acid

As shown in Table 2, collagen powder was added to 0.05M acetic acid and mixed in a blender to form a mixture with either a 1% (standard density) or 2% (double density) solid content (0.55 g or 1.1 g per 100 ml). ORC (0.45 g or 0.90 g per 100 ml) was then added to the mixture and blended to form intermediate slurries. Citric acid was added in appropriate amounts to the intermediate slurries to prepare biomaterial slurries having a citric acid concentration of 100 mM and 200 mM. A portion (31 grams) of each of the biomaterial slurries was transferred into 10 × 10 cm square plates and spread evenly before freezing at - 80°C overnight and followed by freeze drying for 24 hours to prepare a sponge Sample 6 having a citric acid concentration of 100 mM and sponge Sample 7 having a citric acid concentration of 200 mM.

**Table 2**

| **Sponge Samples** | **Collagen Powder and Acetic Acid Mixture** | **ORC** | **Final Citric Acid Concentration (mM)** |
|---|---|---|---|
| 6 | 1% standard density (0.55 g per 100 ml) | 0.45 g per 100 ml | 100 |
| 7 | 2% double density (1.1 g per 100 ml) | 0.90 g per 100 ml | 200 |

### Example 4-Preparation of Biomaterial Film

A biomaterial slurry having a citric acid concentration of 150 mM was prepared as described in Example 1. Glycerol (1.5%) was added to the biomaterial slurry. A portion of the biomaterial slurry was transferred into 10 × 10 cm square plates and spread evenly before being dehydrated for 12-24 hours by a combination of thermal and vacuum dehydration to remove water and produce Film Sample 8.

### Example 5-Biofilm Analysis

### General Methods

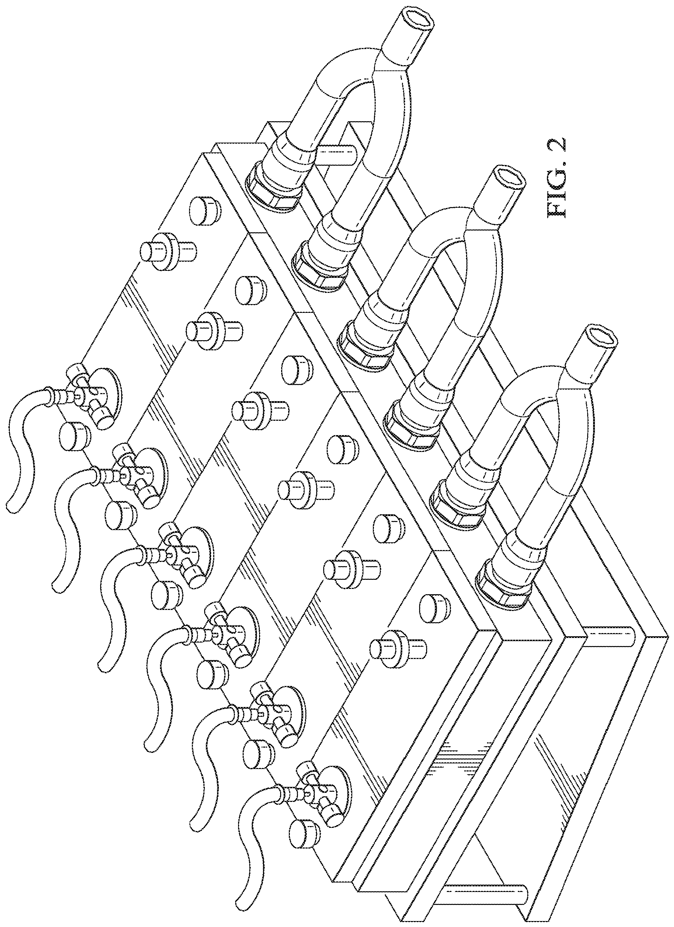

The ability of biomaterials to reduce biofilm populations was investigated using a colony drip flow reactor (C-DFR), based on that described previously by Lipp, C. et al. Testing wound dressings using an in vitro wound model. J Wound Care. 2010;19(6):220-226. To prepare the reactor apparatus, 25mm² absorbent pads (Millipore, Consett, UK) were glued with silicon-based aquarium sealant to clean glass microscope slides and placed in the channels of the C-DFR (Biosurface Technology, Bozeman, MT). The entire set-up was autoclaved and maintained sterile until use. A non-antimicrobial dressing (gauze) was included as a control in each experiment. FIG. 2 shows an example of a C-DFR biofilm model used to grow *Pseudomonas aeruginosa* biofilms as described herein.

Experiments began by hydrating the absorbent pads with 0.5ml of SWF and then 0.22 µm porous polycarbonate membranes (Sigma, Dorset, UK) were placed on these absorbent pads. Next, the membranes were inoculated with 10 µl of a Tryptone Soya broth (TSB)-diluted overnight culture (0.5 McFarland standard suspension). The system was left undisturbed for 30 minutes while the inoculum was allowed to dry. The reactor was then attached to a medium reservoir and SWF was pumped through the system at 5 ml/h/channel. This reactor and set-up allowed the medium to drip down the microscope slide and absorb into the pad, which then supplied nutrients to the bacteria growing on the top side of membrane. The bacteria were then allowed to grow for 72 hours.

After the growth period, one biofilm/membrane per model was subjected to plate counting (see below) to enumerate the biofilm population pre-antimicrobial exposure. For each of the other channels, a sterile sample of biomaterial was placed directly on top of the biofilm/membrane. Dressings were moistened with simulated wound fluid (SWF) to simulate clinical usage. The assay continued for a further 24 hours (flow rate 5 ml/hr/channel), before the dressings were removed and the biofilm/membranes examined with plate counts to enumerate remaining biofilm after antimicrobial exposure. Samples of biofilm/membrane pre-antimicrobial exposure were also subjected to scanning electron microscopy (SEM).

### Plate counting -Enumeration of biofilm

After removal from the C-DFR, biofilm/membranes were rinsed three times with sterile phosphate buffered saline (PBS) to remove any adherent vegetative cells. Samples were added to Dey-Engley neutralising broth to negate any residual antimicrobial effect resulting from dressing contact. The samples were then subjected to 3 minutes of high speed vortexing. Serial 10-fold dilutions were made using sterile Dulbecco's Phosphate Buffered Saline (DPBS), and the dilutions were plated on Trypton Soya Agar (TSA) plates. After 24 hours of incubation at 37°C, the plates were counted and the number of colony forming units (CFU) per membrane was calculated.

### Sample Testing

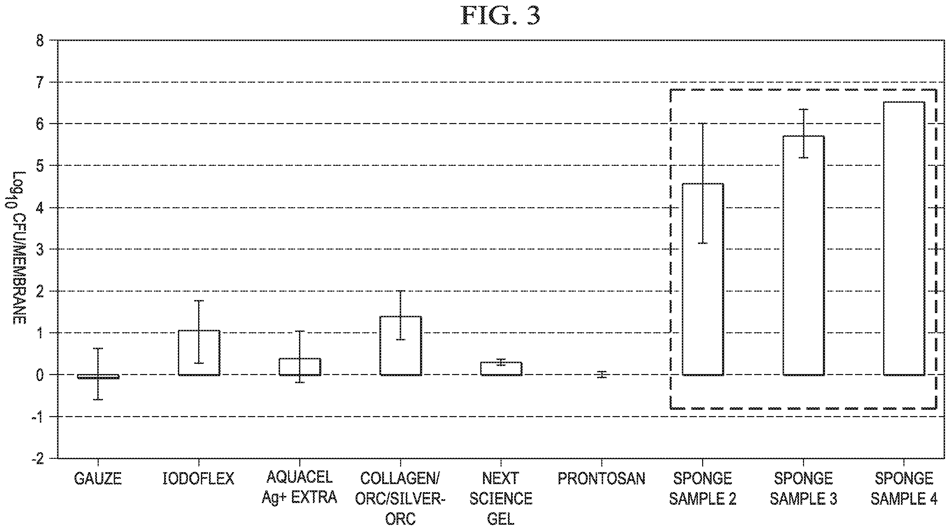

The following samples in Table 3 were tested in the above-described *Pseudomonas aeruginosa* (72 hour old) C-DFR biofilm model and the results are provided in FIGs. 3-6.

**Table 3**

| **Sample** | **Description** |
|---|---|
| Gauze | Topper 8 (Systagenix) |
| Gauze + 100 mM citric acid | 100 mM citric acid solution used to saturate 2.5 × 2.5CM topper 8 gauze prior to application |
| IODOFLEX | Obtained from Smith & Nephew |
| AQUACEL^{®} Ag+ EXTRA^{™} | Obtained from ConvaTec Inc. |
| collagen/ORC/silver-ORC | Prepared according to Example 1 but without the addition of citric acid |
| collagen/ORC/silver-ORC where collagen swelled with 200 mM acetic acid | Prepared as described in Example 2 but using 0.05 M acetic acid and no citric acid |
| NEXT SCIENCE GEL | Obtained from Next Science |
| PRONTONSAN^{®} | Obtained from B. Braun Medical Inc. |
| Sponge Sample 1 | Prepared according to Example 1 |
| Sponge Sample 2 | Prepared according to Example 1 |
| Sponge Sample 3 | Prepared according to Example 1 |
| Sponge Sample 4 | Prepared according to Example 1 |
| collagen/ORC | Prepared according to Example 3 for Sponge Sample 6 but without the addition of citric acid |
| Sponge Sample 6 | Prepared according to Example 3 |
| Sponge Sample 7 | Prepared according to Example |
| Film Sample 8 | Prepared according to Example 4 |

FIG. 3 shows a log reduction of 72 hour old *Pseudomonas aeruginosa* biofilm total viable counts (TVC) compared to T₀ for the following test samples: gauze, IODOFLEX, AQUACEL^{®} Ag+ EXTRA^{™}, collagen/ORC/silver-ORC, NEXT SCIENCE GEL, PRONTONSAN^{®}, Sponge Sample 2, Sponge Sample 3, and Sponge Sample 4. FIG. 3 demonstrates that Sponge Sample 2, Sponge Sample 3, and Sponge Sample 4 achieved the greatest log reduction of 72 hour old *Pseudomonas aeruginosa* biofilm TVC compared to T₀ and thus exhibited superior efficacy in removing biofilms compared to that observed with the other test samples (gauze, IODOFLEX, AQUACEL^{®} Ag+ EXTRA^{™}, collagen/ORC/silver-ORC, NEXT SCIENCE GEL, and PRONTONSAN^{®}).

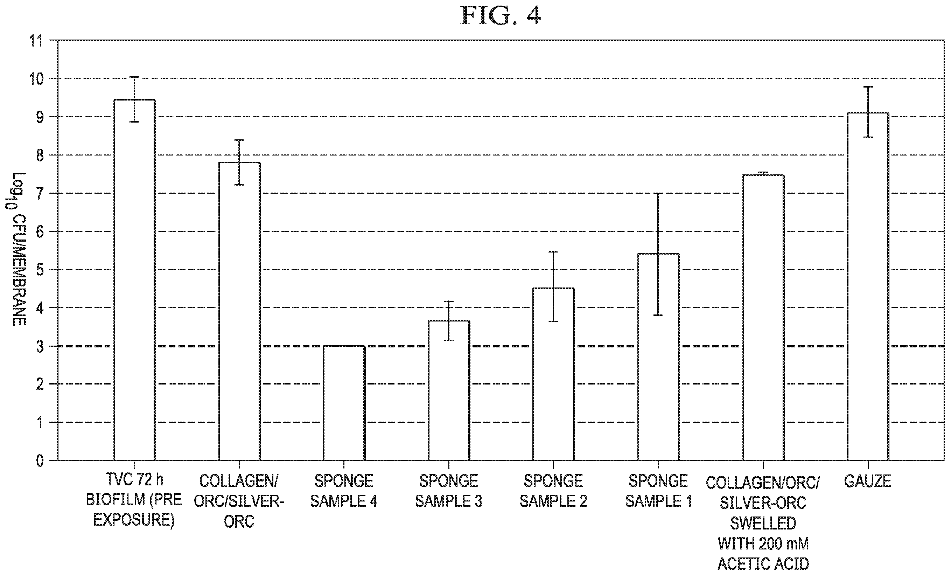

FIG. 4 shows a reduction of 72 hour old *Pseudomonas aeruginosa* biofilm TVC for the following test samples: collagen/ORC/silver-ORC, Sponge Sample 4, Sponge Sample 3, Sponge Sample 2, Sponge Sample 1, and collagen/ORC/silver-ORC swelled with 200 mM acetic acid, and gauze. In FIG. 4, "TVC 72 h biofilm (pre-exposure)" represents the biofilm prior to sample exposure and indicates that *Pseudomonas aeruginosa* biofilm populations reached steady state of ~9.5 log₁₀ units after the 72 hour growth period. *Pseudomonas aeruginosa* biofilm was unaffected by the application of a gauze control dressing during the exposure period, with reductions of less than 1.0 log₁₀ unit observed. Sponge Samples 1-4 showed the largest reduction in *Pseudomonas aeruginosa* biofilm. Sponge Samples 1-4 showed significantly lower biofilm levels compared to the collagen/ORC/silver-ORC and collagen/ORC/silver-ORC swelled with 200 mM acetic acid controls.

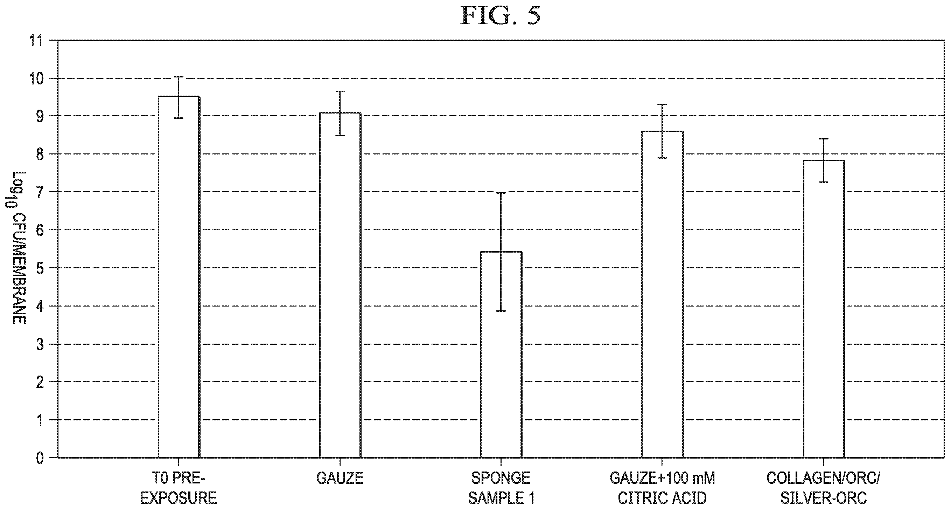

FIG. 5 shows a reduction of 72 hour old *Pseudomonas aeruginosa* biofilm TVC for the following test samples: gauze, Sponge Sample 1, gauze + 100 mM citric acid, and collagen/ORC/silver-ORC. In FIG. 5, "T0 Pre-exposure" represents the biofilm prior to sample exposure and indicates that *Pseudomonas aeruginosa* biofilm populations reached steady state of ~9.5 log₁₀ units after the 72 hour growth period. *Pseudomonas aeruginosa* biofilm was unaffected by the application of a gauze control dressing during the exposure period, with reductions of less than 1.0 log₁₀ unit observed. Sponge Sample 1 showed a synergistic reduction in *Pseudomonas aeruginosa* biofilm compared to that observed with the gauze + 100 mM citric acid, and PROMOGRAN PRISMA^{™} controls.

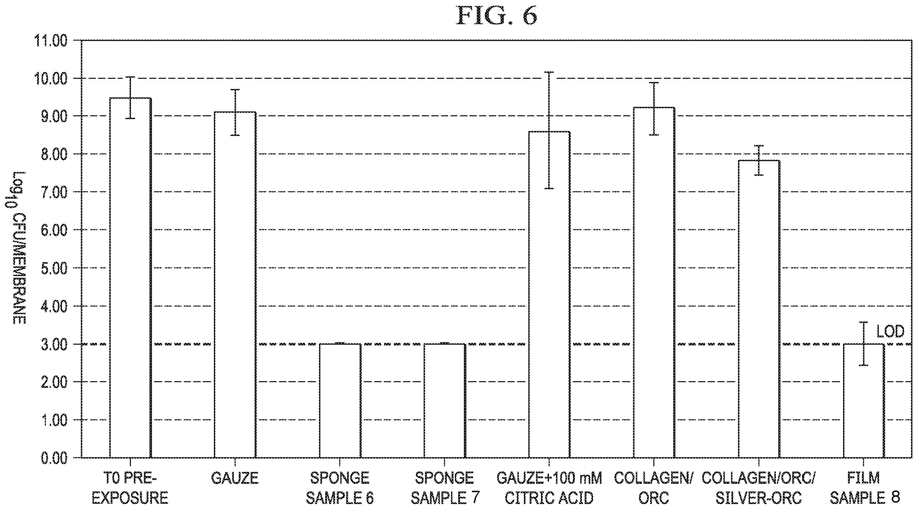

FIG. 6 shows a reduction of 72 hour old *Pseudomonas aeruginosa* biofilm TVC for the following test samples: gauze, Sponge Sample 6, Sponge Sample 7, gauze + 100 mM citric acid, collagen/ORC, collagen/ORC/silver-ORC and Film Sample 8. In FIG. 6, "T0 Pre-exposure" represents the biofilm prior to sample exposure and indicates that *Pseudomonas aeruginosa* biofilm populations reached steady state of ~9.5 log₁₀ units after the 72 hour growth period. *Pseudomonas aeruginosa* biofilm was unaffected by the application of a gauze control dressing and collagen/ORC sponge, with a reduction of less than 1.0 log₁₀ unit observed. Citric acid soaked gauze had a minimal impact on TVC, with a reduction of ~1.0 log₁₀ unit observed. Collagen/ORC/silver-ORC application led to a reduction in biofilm TVC of 1.6 log₁₀ units. All three prototypes (Sponge Sample 6, Sponge Sample 7 and Film Sample 8) reduced biofilm populations to below detection limits (>6.0 log₁₀ unit reductions) after 24 hour continuous exposure and exhibited synergistic effects compared to that observed with gauze + 100 mM citric acid controls.

These results demonstrate that the biomaterials of the present technology are useful in methods for preventing, reducing, inhibiting or removing biofilms as well as treating wounds in a subject in need thereof.

### Example 6-Cytoxicity Analysis

An intermediate slurry was prepared according to Example 1. Citric acid in the amounts provided in Table 3 was solubilized in 5 ml of water and added to 120 ml of the intermediate slurry to prepare biomaterial slurries having varying citric acid concentrations. A portion (31 grams) of each of the biomaterial slurries with varying citric acid concentrations were transferred into 10 × 10 cm square Petri dishes and spread evenly before freezing at -80°C overnight and followed by freeze drying for 24 hours to prepare sponge Samples 9, 10, and 11 having a citric acid concentration of 100 mM, 150 mM, and 200 mM, respectively.

**Table 4**

| **Sponge Samples** | **Final Citric Acid Concentration (mM)** | **Citric Acid Amount Added (g/120 ml)** |
|---|---|---|
| 9 | 100 | 2.295 |
| 10 | 150 | 3.44 |
| 11 | 200 | 4.661 |

The biological response of mammalian cells after exposure to sponge Samples 9, 10 and 11 (in triplicate) was assessed according to ISO-10993-5 2009 (Cytotoxicity by Indirect Agar Diffusion). The numerical grading of cytotoxicity used is provided below in Table 5.

**Table 5**

| **Grade** | **Interpretation** | **Conditions of All Cultures** |
|---|---|---|
| 0 | Non-cytotoxic | No detectable zone around or under specimen |
| 1 | Slightly cytotoxic | Some malformed or degenerated cells under specimen |
| 2 | Mildly cytotoxic | Zone limited to area under specimen |
| 3 | Moderately cytotoxic | Zone extending specimen size up to 1 cm |
| 4 | Severely cytotoxic | Zone extends > 1.0 cm beyond specimen |

The results of the test are shown below in Table 6. All samples tested were determined to be Grade 0 in the assay (no cytotoxicity).

**Table 6**

| **Sample** | **Replicate** | **Cytotoxicity Grade** | **Reactivity** |
|---|---|---|---|
| Sponge Sample 9 | 1 | 0 | Non -cytotoxic |
| Sponge Sample 9 | 2 | 0 | Non -cytotoxic |
| Sponge Sample 9 | 3 | 0 | Non -cytotoxi c |
| Sponge Sample 10 | 1 | 0 | Non -cytotoxi c |
| Sponge Sample 10 | 2 | 0 | Non -cytotoxi c |
| Sponge Sample 10 | 3 | 0 | Non -cytotoxi c |
| Sponge Sample 11 | 1 | 0 | Non -cytotoxi c |
| Sponge Sample 11 | 2 | 0 | Non -cytotoxi c |
| Sponge Sample 11 | 3 | 0 | Non -cytotoxi c |

These results demonstrate that the biomaterials of the present technology are useful in methods for preventing, reducing, inhibiting or removing biofilms as well as treating wounds in a subject in need thereof.

"Include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Moreover, descriptions of various alternatives using terms such as "or" do not require mutual exclusivity unless clearly required by the context, and the indefinite articles "a" or "an" do not limit the subject to a single instance unless clearly required by the context.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if parameter X is exemplified herein to have values in the range of 1 - 10, or 2 - 9, or 3 - 8, it is also envisioned that Parameter X may have other ranges of values including 1 - 9, 1 - 8, 1 - 3, 1 - 2, 2 - 10, 2 - 8, 2 - 3, 3 - 10, and 3 - 9.

"About" is intended to refer to deviations in a numerical quantity that may result from various circumstances, for example, through measuring or handling procedures in the real world; through inadvertent error in such procedures; through differences in the manufacture, source, or purity of compositions or reagents; from computational or rounding procedures; and other deviations as will be apparent by those of skill in the art from the context of this disclosure. For example, the term "about" may refer to deviations that are greater or lesser than a stated value or range by 1/10 of the stated value(s), e.g., ±10%, as appropriate from the context of the disclosure. For instance, a concentration value of "about 30%" may refer to a concentration between 27% and 33%. Whether or not modified by the term "about," quantitative values recited in the claims include equivalents to the recited values, for example, deviations from the numerical quantity, as would be recognized as equivalent by a person skilled in the art in view of this disclosure.

## Claims

1. A biomaterial comprising collagen, citric acid and oxidized regenerated cellulose (ORC) for use in preventing, reducing, inhibiting, disrupting or removing a biofilm present in a wound site.

2. The biomaterial for use of claim 1, wherein the ORC is present in an amount of about 25 wt% to about 65 wt% based on the total weight of the biomaterial, or about 40 wt% to about 50 wt% based on the total weight of the biomaterial.

3. The biomaterial for use of any one of the previous claims, further comprising silver.

4. The biomaterial for use of claim 3, wherein at least a portion of the silver is present as an ORC-silver complex, optionally wherein the ORC-silver complex is present in an amount of about 0.10 wt% to about 3.0 wt% based on the total weight of the biomaterial, or about 0.50 wt% to about 5.0 wt% based on the total weight of the biomaterial.

5. The biomaterial for use of any one of the previous claims, wherein the collagen is present in an amount of about 35 wt% to about 75 wt% based on the total weight of the biomaterial, or about 50 wt% to about 60 wt% based on the total weight of the biomaterial.

6. The biomaterial for use of any one of the previous claims, wherein the citric acid is present in a concentration ≥ about 20 mM, optionally in a concentration of about 20 mM to about 600 mM, or about 20 mM to about 400 mM.

7. The biomaterial for use of any one of the previous claims, wherein the biomaterial reduces the biofilm by about ≥ 2 log₁₀ units or by about ≥ about 3 log₁₀ units after 24 hours *in vitro* exposure.

8. The biomaterial for use of any one of the previous claims, wherein the biomaterial further comprises perforations.

9. The biomaterial for use of any one of the previous claims, wherein the biomaterial is in the form of a sponge, a film, a foam, a gel, a bead, a rope, a polymeric matrix, a coating, or a solution.

10. The biomaterial for use of any one of claims 1 to 8, wherein the biomaterial is in the form a film.

11. The biomaterial for use of claim 10, wherein the biomaterial further comprises glycerol.

12. The biomaterial for use of claim 10 or 11, wherein the film is flexible or rigid.

13. The biomaterial for use of any one of claims 10 to 12, wherein the film is substantially transparent.

14. The biomaterial for use of any one of claims 10 to 13, wherein the film comprises a grid.

15. The biomaterial for use of any one of claims 1 to 8, wherein the biomaterial is in the form of a sponge.

16. The biomaterial for use of any one of claims 1 to 8, wherein the biomaterial is in the form of a foam.

17. The biomaterial for use of claim 1, wherein said use further comprises negative pressure wound therapy, optionally further comprising sealing the biomaterial to tissue surrounding the wound site to form a sealed space, optionally further comprising:
fluidly coupling a negative-pressure source to the sealed space; and
operating the negative-pressure source to generate a negative pressure in the sealed space.

## Patentansprüche

1. Biomaterial, umfassend Kollagen, Zitronensäure und oxidierte regenerierte Zellulose (ORC) zur Verwendung bei der Verhinderung, Reduzierung, Hemmung, Unterbrechung oder Entfernung eines Biofilms, der in einer Wundstelle vorhanden ist.

2. Biomaterial zur Verwendung nach Anspruch 1, wobei die ORC in einer Menge von etwa 25 Gew.-% bis etwa 65 Gew.-%, basierend auf dem Gesamtgewicht des Biomaterials, oder etwa 40 Gew.-% bis etwa 50 Gew.-%, basierend auf dem Gesamtgewicht des Biomaterials, vorhanden ist.

3. Biomaterial zur Verwendung nach einem der vorstehenden Ansprüche, weiter umfassend Silber.

4. Biomaterial zur Verwendung nach Anspruch 3, wobei mindestens ein Teil des Silbers als ein ORC-Silberkomplex vorhanden ist, wobei der ORC-Silberkomplex optional in einer Menge von etwa 0,10 Gew.-% bis etwa 3,0 Gew.-%, basierend auf dem Gesamtgewicht des Biomaterials, oder etwa 0,50 Gew.-% bis etwa 5,0 Gew.-%, basierend auf dem Gesamtgewicht des Biomaterials, vorhanden ist.

5. Biomaterial zur Verwendung nach einem der vorstehenden Ansprüche, wobei das Kollagen in einer Menge von etwa 35 Gew.-% bis etwa 75 Gew.-%, basierend auf dem Gesamtgewicht des Biomaterials, oder etwa 50 Gew.-% bis etwa 60 Gew.-%, basierend auf dem Gesamtgewicht des Biomaterials, vorhanden ist.

6. Biomaterial zur Verwendung nach einem der vorstehenden Ansprüche, wobei die Zitronensäure in einer Konzentration von ≥ etwa 20 mM, optional in einer Konzentration von etwa 20 mM bis etwa 600 mM oder etwa 20 mM bis etwa 400 mM vorhanden ist.

7. Biomaterial zur Verwendung nach einem der vorstehenden Ansprüche, wobei das Biomaterial den Biofilm nach 24 Stunden *In-vitro*-Exposition um etwa ≥ 2 log₁₀ Einheiten oder um etwa ≥ 3 log₁₀ Einheiten reduziert.

8. Biomaterial zur Verwendung nach einem der vorstehenden Ansprüche, wobei das Biomaterial weiter Perforationen umfasst.

9. Biomaterial zur Verwendung nach einem der vorstehenden Ansprüche, wobei das Biomaterial in der Form eines Schwamms, eines Films, eines Schaums, eines Gels, einer Wulst, eines Seils, einer Polymermatrix, einer Beschichtung oder einer Lösung vorliegt.

10. Biomaterial zur Verwendung nach einem der Ansprüche 1 bis 8, wobei das Biomaterial in der Form eines Films vorliegt.

11. Biomaterial zur Verwendung nach Anspruch 10, wobei das Biomaterial ferner Glycerol umfasst.

12. Biomaterial zur Verwendung nach Anspruch 10 oder 11, wobei der Film flexibel oder starr ist.

13. Biomaterial zur Verwendung nach einem der Ansprüche 10 bis 12, wobei der Film im Wesentlichen transparent ist.

14. Biomaterial zur Verwendung nach einem der Ansprüche 10 bis 13, wobei der Film ein Gitter umfasst.

15. Biomaterial zur Verwendung nach einem der Ansprüche 1 bis 8, wobei das Biomaterial in der Form eines Schwamms vorliegt.

16. Biomaterial zur Verwendung nach einem der Ansprüche 1 bis 8, wobei das Biomaterial in der Form eines Schaums vorliegt.

17. Biomaterial zur Verwendung nach Anspruch 1, wobei die Verwendung weiter Unterdruck-Wundtherapie umfasst, optional ferner umfassend ein Abdichten des Biomaterials gegenüber Gewebe, das die Wundstelle umgibt, um einen abgedichteten Bereich auszubilden, optional ferner umfassend:
fluidisches Koppeln einer Unterdruckquelle an den abgedichteten Bereich; und
Betreiben der Unterdruckquelle, um in dem abgedichteten Bereich einen Unterdruck zu erzeugen.

## Revendications

1. Biomatériau comprenant du collagène, de l'acide citrique et de la cellulose régénérée oxydée (CRO) pour une utilisation dans la prévention, la réduction, l'inhibition, la perturbation ou l'élimination d'un biofilm présent dans un site de plaie.

2. Biomatériau pour une utilisation selon la revendication 1, dans lequel la CRO est présente en une quantité d'environ 25 % en poids à environ 65 % en poids sur la base du poids total du biomatériau, ou d'environ 40 % en poids à environ 50 % en poids sur la base du poids total du biomatériau.

3. Biomatériau pour une utilisation selon l'une quelconque des revendications précédentes, comprenant en outre de l'argent.

4. Biomatériau pour une utilisation selon la revendication 3, dans lequel au moins une partie de l'argent est présente sous la forme d'un complexe CRO-argent, optionnellement dans lequel le complexe CRO-argent est présent en une quantité d'environ 0,10 % en poids à environ 3,0 % en poids sur la base du poids total du biomatériau, ou d'environ 0,50 % en poids à environ 5,0 % en poids sur la base du poids total du biomatériau.

5. Biomatériau pour une utilisation selon l'une quelconque des revendications précédentes, dans lequel le collagène est présent en une quantité d'environ 35 % en poids à environ 75 % en poids sur la base du poids total du biomatériau, ou d'environ 50 % en poids à environ 60 % en poids sur la base du poids total du biomatériau.

6. Biomatériau pour une utilisation selon l'une quelconque des revendications précédentes, dans lequel l'acide citrique est présent en une concentration ≥ à environ 20 mM, optionnellement en une concentration d'environ 20 mM à environ 600 mM, ou d'environ 20 mM à environ 400 mM.

7. Biomatériau pour une utilisation selon l'une quelconque des revendications précédentes, dans lequel le biomatériau réduit le biofilm d'environ ≥ 2 unités log₁₀ ou d'environ ≥ 3 unités log₁₀ après une exposition de 24 heures *in vitro.*

8. Biomatériau pour une utilisation selon l'une quelconque des revendications précédentes, dans lequel le biomatériau comprend en outre des perforations.

9. Biomatériau pour une utilisation selon l'une quelconque des revendications précédentes, dans lequel le biomatériau est sous la forme d'une éponge, d'un film, d'une mousse, d'un gel, d'une bille, d'une corde, d'une matrice polymère, d'un revêtement ou d'une solution.

10. Biomatériau pour une utilisation selon l'une quelconque des revendications 1 à 8, dans lequel le biomatériau est sous la forme d'un film.

11. Biomatériau pour une utilisation selon la revendication 10, dans lequel le biomatériau comprend en outre du glycérol.

12. Biomatériau pour une utilisation selon la revendication 10 ou 11, dans lequel le film est souple ou rigide.

13. Biomatériau pour une utilisation selon l'une quelconque des revendications 10 à 12, dans lequel le film est sensiblement transparent.

14. Biomatériau pour une utilisation selon l'une quelconque des revendications 10 à 13, dans lequel le film comprend une grille.

15. Biomatériau pour une utilisation selon l'une quelconque des revendications 1 à 8, dans lequel le biomatériau est sous la forme d'une éponge.

16. Biomatériau pour une utilisation selon l'une quelconque des revendications 1 à 8, dans lequel le biomatériau est sous la forme d'une mousse.

17. Biomatériau pour une utilisation selon la revendication 1, dans lequel ladite utilisation comprend en outre une thérapie de plaie par pression négative, comprenant en outre optionnellement l'application étanche du biomatériau sur le tissu entourant le site de plaie pour former un espace étanche, comprenant en outre optionnellement :
le couplage fluidique d'une source de pression négative à l'espace étanche ; et
le fonctionnement de la source de pression négative pour générer une pression négative dans l'espace étanche.
